# EUROPEAN PATENT APPLICATION

(11) **EP 1 084 731 A1**
(43) Date of publication of application: **21.03.2001**
(21) Application number: 99921346.5
(22) Date of filing: 17.02.1999
(51) Int. Cl.: A61N 5/00, A01G 7/00

(54) **A. V. KRYLOV'S ACTIVATOR**

(71) Applicant: Krylov, Valery Alexeevich, Odessa, 270104 (UA)
(72) Inventor: Krylov, Valery Alexeevich, Odessa, 270104 (UA)
(74) Representative: Radwer, Dieter
(86) International application number: UA9900003
(87) International publication number: WO0048671

(57) **Zusammenfassung**

Die vorliegende Erfindung betrifft eine Vorrichtung für die biologische Aktivierung von Wasser, Boden, Pflanzen, landwirtschaftlichen Saatguts und anderer biogener Objekte unter Verwendung natürlicher Minerale.

Der vorliegende Aktivator enthält eine Auswahl von aktivierenden Elementen mit unterschiedlicher Form und Farbe. Jedes aktivierende Element besteht aus pontischem Kalkstein und ist hergestellt in Form eines Körpers, der durch zwei parallele Geraden gebildet wird, die eine Fläche bilden, die in einem Winkel von 90° zur Bezugslinie der geologischen Schicht des Minerals geneigt ist. Die aktivierenden Elemente sind auf einer Unterlage aus einem metallfreien Werkstoff fest verbunden. Die Stärke jedes aktivierenden Elements kann unterschiedlich sein und beträgt mindestens 3 mm. Das Verhältnis der Gesamtfläche der aktivierenden Elemente und der Fläche der Unterlage ist gleich 0,75 -1,0. Die aktivierenden Elemente sind auf der Unterlage befestigt und in einem durchsichtigen Gehäuse angeordnet. Der Aktivator besitzt einen hohen Aktivierungsgrad auf Wasser, Boden, Pflanzen und andere Objekte

## Description

Die Erfindung betrifft eine Vorrichtung für die biologische Aktivierung von Wasser, Boden, Pflanzen, Samen landwirtschaftlicher Kulturen u.a. biogener Objekte mit Heilwirkung auf den Menschen mittels natürlicher Mineralien.

Bekanntlich sind in jedem konkreten natürlichen Mineral eine bestimmte Auswahl von Elementen oder ein einzelnes Element des Periodensystems enthalten. So wurden beispielsweise in Korallen Prostaglandine 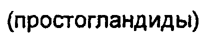 entdeckt; Stoffe, die unter natürlichen Bedingungen an der Regulierung vieler Lebensfunktionen des menschlichen Organismus beteiligt sind: wie beispielsweise des Atmungs- und Verdauungssystems, des Blutkreislaufs oder der Fortpflanzung. Folglich wirken die verschiedenen Mineralien auf unterschiedliche Weise auch auf Wasser, Boden, Mensch und Pflanzen.

Das Gesagte belegt, daß der Mensch seit Jahrtausenden natürliche Mineralien (Amazonenstein, Malachit, Lasurit, Mondstein, Bergkristall, Jaspis, Amethyst, Bernstein und viele andere) zur Heilung von Krankheiten (Erkrankungen des zentralen und peripheren Nervensystems, Kopfschmerzen, Migräne, Schlaflosigkeit, akuter und chronischer Erkrankungen des Magen-Darm-Trakts und vieler anderer) und für Dekorationszwecke verwendet.

Ferner ist bekannt, daß einige Mineralien aktivierende (tonisierende) Eigenschaften besitzen, wie Granat und Rubin.

Auf der Basis der natürlichen Mineralien wurden verschiedene aktivierende Elemente ausgewählt, die dem Wasser eine biologische Aktivität verleihen. Biologisch aktives Wasser wird in der Medizin für die Prophylaxe und Heilung von Erkrankungen des Magen-Darm-Trakts, der Nieren und des Urogenitalsystems verwendet. Gute Ergebnisse wurden auch in der Züchtung und in der Saatzucht sowie in der Zierpflanzenzucht erzielt.

Von den bekannten Vorrichtungen, die der Erfindung am naheliegendsten sind, ist eine Vorrichtung für die biologische Wasseraktivierung zu nennen, die aus einem Glasgefäß und aktivierenden Elementen verschiedener geometrischer Form aus einem polierten natürlichen Mineral - Silizium - besteht. Die aktivierenden Elemente sind in einem mit Wasser gefüllten Gefäß angeordnet (s. Gonikman E.I., Ihr Talismann, Verlag "Santana", Minsk, 1992, S. 86-91).

Diese Lösung ist als Prototyp (der Erfindung am nächsten stehend) anzusehen. Die angemeldete erfindungsgemäße Lösung hat mit dem Prototypen gemeinsam, daß beide aktivierende Elemente verschiedener Form aus einem natürlichen Mineral verwenden. Jedoch bestehen in der bekannten Vorrichtung die aktivierenden Elemente aus Silizium, besitzen eine willkürliche (nichtfestgelegte) Form, sind stark poliert und befinden sich in dem Glasgefäß direkt im Wasser. Außerdem können die aktivierenden Elemente in der bekannten Vorrichtung monochrom, d.h. einfarbig sein und sind nicht auf einer Unterlage (Träger) befestigt, sondern befinden sich einfach poliert in ihrem natürlichen Zustand in Wasser.

Die Besonderheiten des Minerals, aus denen die aktivierenden Elemente (Silizium) bestehen, ihre Form (willkürlich) und die anderen genannten Eigenschaften ermöglichen es nicht, dem Wasser eine hohe biologische Aktivität zu verleihen. Demzufolge ist die Heilkraft eines solchen Wassers nicht sehr bedeutsam, und es hat eher eine psychologische als therapeutische Wirkung auf den Menschen. Dies wird durch vergleichende Untersuchungen bestätigt, wie sie in der Beschreibung der Erfindung nachstehend dargelegt werden.

Der Erfindung liegt die Aufgabe zugrunde, einen Aktivator zu schaffen, bei dem durch Änderung der Form der aktivierenden Elemente des Stoffes, aus dem sie bestehen, der festgelegten Farbe und der Befestigung derselben auf einer bestimmten Unterlage (Träger) mit vorgegebener Stärke ein hoher aktiver Wirkungsgrad auf Mensch, Wasser, Boden, Pflanzen, Samen landwirtschaftlicher Kulturen und andere Objekte der Umwelt gewährleistet ist.

Die gestellte Aufgabe wird durch einem Aktivator gelöst, der eine Auswahl von aktivierenden Elementen verschiedener Form aus einem natürlichen Mineral enthält, wobei im Unterschied zu der bekannten Vorrichtung jedes aktivierende Element aus verschiedenfarbigem pontischem Kalkstein in Form eines Körpers besteht, der durch zwei parallele Geraden gebildet wird, die eine Fläche mit einem Winkel von 90 ° zur Bezugslinie der geologischen Schicht des Minerals bilden, wobei die aktivierenden Elemente mit einer Unterlage fest verbunden sind, die kein Metall enthält.

Ferner beträgt das Verhältnis der Gesamtfläche der aktivierenden Elemente zur Fläche der Unterlage, auf der sie befestigt sind, 0,75-1,0.

Die auf der Unterlage befestigten aktivierenden Elemente sind in einem durchsichtigen Gehäuse aus einem nicht mit einem Metall beschichteten Werkstoff angeordnet.

Neu am angemeldeten Aktivator sind:
- der Stoff (Mineral), aus dem die aktivierenden Elemente bestehen, ist pontischer Kalkstein;
- die Verschiedenfarbigkeit der aktivierenden Elemente, d.h. Monochromasie ist unzulässig;
- die Form der Ausführung jedes aktivierenden Elements - es ist ein Körper, der durch zwei parallele Geraden gebildet wird, die eine Fläche in einem Winkel von 90 ° zur Bezugslinie der geologischen Schicht des Minerals bilden;
- die feste Verbindung mehrerer aktivierender Elemente auf der Unterlage;
- die Ausführung der Unterlage aus einem Stoff, der kein Metall enthält.

Weitere Neuheitsmerkmale der Erfindung sind:
- das Verhältnis der Gesamtfläche der aktivierenden Elemente zur Fläche der Unterlage, auf der sie befestigt sind, ist gleich 0,75-1,0;
- die Anordnung der auf der Unterlage befestigten aktivierenden Elemente in einem durchsichtigen Gehäuse.

Die Stärke jedes aktivierenden Elements kann verschieden sein, sollte jedoch nicht unter 3 mm liegen.

Nachstehend wird der Zusammenhang zwischen den angemeldeten Neuheitsmerkmalen und dem erzielten Ergebnis erläutert.

Die Struktur von Silizium und pontischem Kalkstein ist verschieden. Pontischer Kalkstein besitzt eine komplizierte Resonanzstruktur, die durch die Kristallgröße und Mikrorisse entsteht, in denen eine große Perlmuttstoffmenge enthalten ist Perlmutt besteht aus Kalziumkarbonat sowie aus einer geringen Menge Strontium und anderen Elementen.

Man kann annehmen, daß die genannte Struktur und die Form der Ausführung der aktivierenden Elemente die biologische Aktivität und Heilkraft des behandelten Leitungswassers erhöhen. Die Ausführungsform der aktivierenden Elemente, das Verhältnis der Gesamtfläche der aktivierenden Elemente zur Fläche der Unterlage, auf der sie befestigt sind, wurde experimentell ermittelt.

Die Ausführung der aktivierenden Elemente aus verschiedenfarbigen umkristallisierten Kalkstein des Jungtertiärs (pontischem Kalkstein) sowie die feste Verbindung derselben auf der Unterlage in geringem Abstand voneinander verstärkt das Resonanzsystem des Aktivators, wenn die aktivierenden Elemente Schall und Licht reflektieren.

Die Verschiedenfarbigkeit der Minerale, die auf die unterschiedliche Struktur (verschiedene Kristall- und Mikrorißgröße) zurückzuführen ist, gewährleistet die unterschiedliche Wellenlänge der reflektierten Signale und erzeugt so ein Pulsieren des Kraftfeldes der aus pontischem Kalkstein bestehenden aktivierenden Elemente.

Die Erfindung soll nahstehend an einem Ausführungsbeispiel näher erläutert werden. In den dazugehörigen Zeichnungen zeigen:

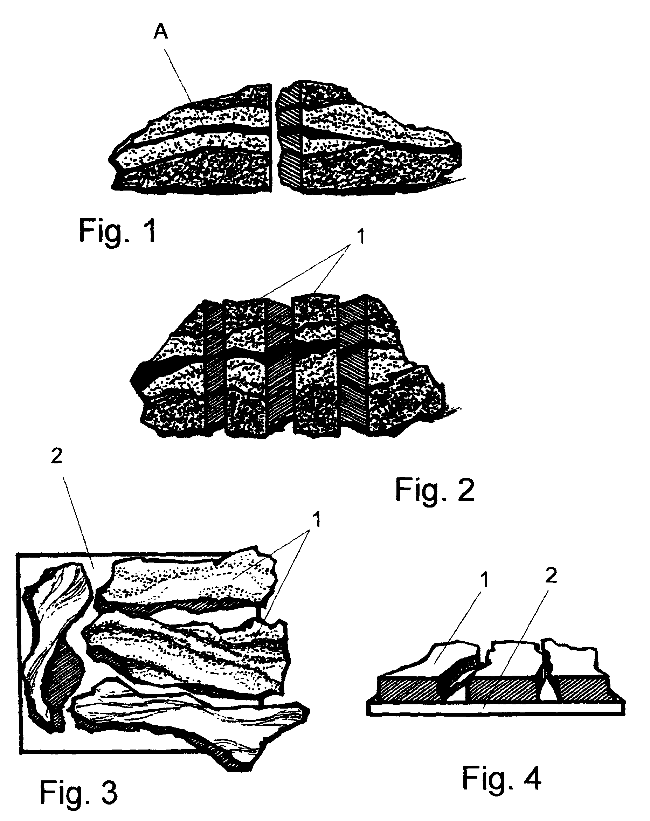

- Fig. 1: eine Ansicht des pontischen Kalksteins als Meerespellet mit einer Schnittfläche im Winkel von 90° zur Bezugslinie der geologischen Schicht (A),
- Fig. 2: eine Ansicht der aktivierenden Elemente, die durch Trennung eines monolithischen Minerals gewonnen wurden,
- Fig. 3: eine Draufsicht der aktivierenden Elemente, die mit einer Unterlage fest verbunden sind,
- Fig. 4: eine Seitenansicht der mit der Unterlage fest verbundenen aktivierenden Elemente,
- Fig. 5: zeigt den Aktivator, auf dem ein Glasgefäß mit Wasser steht;
- Fig. 6: zeigt die mit der Unterlage fest verbundenen aktivierenden Elemente in einem durchsichtigen Gehäuse,
- Fig. 7: zeigt den Aktivator, auf dem sich ein Blumentopf mit einer Pflanze befindet.

Der Aktivator enthält eine Auswahl von aktivierenden Elementen 1, die aus verschiedenfarbigen pontischem Kalkstein bestehen. Jedes aktivierende Element 1 ist als ein Körper ausgebildet, der durch zwei parallele Geraden gebildet wird, die eine Fläche, die in einem Winkel von 90° zur Bezugslinie der geologischen Schicht A des Minerals geneigt ist, bilden. Die aktivierenden Elemente 1 sind fest mit der Unterlage 2 verbunden. Die Stärke jedes aktivierenden Elements 1 beträgt mindestens 3 mm. Das Verhältnis der Gesamtfläche der aktivierenden Elemente 1 zur Fläche der Unterlage 2 beträgt 0,75 - 1,0.

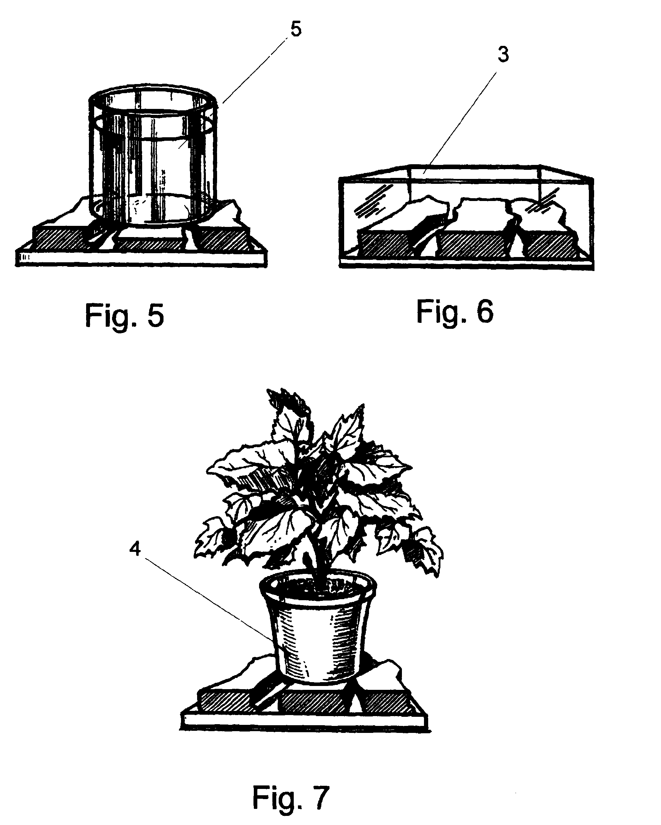

Zur Einwirkung auf eine größere Wassermenge, die sich beispielsweise in einem Wasserbecken befindet, können die aktivierenden Elemente 1, die auf der Unterlage 2 befestigt sind, in einem Gehäuse 3 aus durchsichtigem Material (Fig. 6) angeordnet sein, das keine metallischen Einschlüsse enthält.

Zur Einwirkung auf eine Pflanze stellt man auf die aktivierenden Elemente 1 einen Topf 4 mit Blumen (Fig. 7).

Zur Einwirkung auf Wasser in kleineren Behältnissen stellt man auf die aktivierenden Elemente 1 ein Glasgefäß 5, das mit Wasser gefüllt ist (Fig. 5).

Die Funktionsweise und die Wirkung des erfindungsgemäßen Aktivators wurden an verschiedenen Objekten erprobt.

Die Aktivierung von Trinkwasser geschieht folgendermaßen:
Das Glasgefäß 5 füllt man mit Leitungswasser in einem dünnen Strahl und stellt es auf die aktivierenden Elemente 1, die fest mit der Unterlage 2 verbunden sind. Nach 6 Stunden gelangt das Wasser in einen Zustand biologischer Aktivität. Es ist nun verabreichbar. Die organoleptische Bewertung des biologisch aktiven Wassers ergab, daß es in seinen Trinkeigenschaften weicher ist und Quellwasser nahekommt.

Die Einwirkung auf Pflanzen erfolgt entweder ähnlich der Aktivierung von Wasser, jedoch stellt man den Pflanzentopf 4 auf die fest mit der Unterlage 2 verbundenen aktivierenden Elemente 1, oder besprengt die Pflanzen regelmäßig mit dem biologisch aktiven Wasser, das mit dem Aktivator, wie oben beschrieben, behandelt wurde.

Es wurden die Eigenschaften des biologisch aktiven Wassers untersucht (in Gewächshäusern des Sanatoriums "Rossija" in Odessa), das mit dem Aktivator mit unterschiedlichen aktivierenden Elementen behandelt worden war.
Beispiel 1 - die aktivierenden Elemente bestanden aus Marmor. Der Schnittwinkel zur Bezugslinie der geologischen Schicht betrug 90°. Das Verhältnis der Gesamtfläche der aktivierenden Elemente 1 zur Fläche der Unterlage 2 betrug 1,0.
Beispiel 2 - die aktivierenden Elemente bestanden aus pontischem Kalkstein. Der Schnittwinkel zur Bezugslinie der geologischen Schicht betrug 0°. Das Verhältnis der Gesamtfläche der aktivierenden Elemente 1 zur Fläche der Unterlage betrug 1,0.
Beispiel 3 - die aktivierenden Elemente 1 bestanden aus dem gleichen Mineral wie in Beispiel 2, jedoch wurde ein Schnittwinkel von 30° gewählt. Das Verhältnis der Bezugsflächen war das gleiche wie in Beispiel 1 und 2.
Beispiel 4 - die aktivierenden Elemente 1 bestanden aus dem gleichen Mineral wie in Beispiel 2, der Schnittwinkel betrug jedoch 90°. Das Verhältnis der Bezugsflächen war das gleiche wie in Beispiel 1, 2 und 3.
Beispiel 5 - die aktivierenden Elemente 1 bestanden aus dem gleichen Mineral wie in Beispiel 2, 3, 4, der Schnittwinkel betrug 90°, das Verhältnis der Gesamtfläche der aktivierenden Elemente 1 zur Fläche der Unterlage 2 betrug jedoch 0,75.
Beispiel 6 unterscheidet sich von Beispiel 5 dadurch, daß das Verhältnis der Gesamtfläche der aktivierenden Elemente 1 zur Fläche der Unterlage 2 0,6 betrug.
Beispiel 7 - das Wasser wurde so behandelt wie die der Erfindung naheliegendste Lösung. Die aktivierenden Elemente bestanden aus Silizium und waren in einem Glasbehälter angeordnet, der mit Leitungswasser gefüllt war. Die biologische Aktivität des Wassers wurde an Mohrrübensamen der Sorte Nantskaya 1994 mit einer allgemein bekannten schlechten Keimfähigkeit getestet. Hierzu wurden 9 Petri-Schalen mit Filterpapier auf dem Boden verwendet, das mit den verschiedenen Wasserproben getränkt war (Beispiele 1 - 7). Auf das befeuchtete Papier wurden in jede Schale je 100 Samenkerne gegeben und diese 10 Tage lang behandelt, indem regelmäßig das Papier mit den Wasserproben befeuchtet wurde. Anschließend wurde die Zahl der aufgegangenen Samenkorne gezählt. Die Ergebnisse sind in der Tabelle angeführt. Wie aus der Tabelle ersichtlich ist, weisen die Beispiele 4 und 5 die besten Keimungsergebnisse auf.
   Der erfindungsgemäße Aktivator wurde für die Behandlung von Harnstein-Erkrankungen eingesetzt.
Beispiel 8. Der Patient P., 56 Jahre. Innerhalb von 3 Jahren hatte er Kuraufenthalte zur Heilung einer fortschreitenden Harnstein-Erkrankung. Am 10.10.1996 wurde ein chirurgischer Eingriff an ihm zur Zertrümmerung und Abführung eines 8 mm großen Harnsteins aus der rechten Niere vorgenommen. Nach 4 Monaten bildete sich erneut ein Harnstein in der rechten Niere. Innerhalb von 2 Monaten erreichte er eine Größe von 8 mm. Seit dem 01.03.1997 trank der Patient täglich das mit dem erfindungsgemäßen Aktivator behandelte Wasser.
   Nach Ablauf von 30 Tagen ergab die klinische Untersuchung, daß keine Harnsteine mehr in den Nieren anzutreffen waren. Seit April 1997 bis heute sind keine neuen Harnsteinbildungen zu beobachten.
Beispiel 9. Der Patient K., 69 Jahre. Die rechte Niere war entfernt, in der linken Niere befanden sich mehrere Steine mit einer Größe von 3 bis 10 mm. Dem Patienten wurde die tägliche Zusichnahme von Wasser auf nüchternen Magen verordnet, das mit dem erfindungsgemäßen Aktivator behandelt war. Am 13. Tage der Zusichnahme des aktivierten Wassers begannen die Steine als Grieß abzugehen. Aller 5-6 Tage mit einer Unterbrechung von 2-3 Tagen innerhalb von 2 Monaten ging Grieß ab. In den darauffolgenden 7 Monaten war eine neue Harnsteinbildung nicht festzustellen.

Der erfindungsgemäße Aktivator wurde auch für die biologische Aktivierung von Wasser getestet, das für die Behandlung von Patienten mit Magen-Darm-Krankheiten im 411. Militärbezirks-Krankenhaus eingesetzt wurde.

Beobachtet wurden 20 Patienten, die neben der traditionellen Behandlung biologisch aktives Wasser tranken, und 20 Testpatienten, die normales abgekochtes Wasser zu sich nahmen. In 85 % der Fälle verbesserte sich bei der ersten Gruppe um zwei Tage früher als bei der Kontrollgruppe ihr subjektives Befinden, verringerten sich die Durchfallbeschwerden, Blähungen und das Kollern im Darmtrakt.

Über die Ergebnisse der Erprobung des biologisch aktiven Wassers wurde ein Gutachten über dessen Wirksamkeit bei der Behandlung von Erkrankungen des Magen-Darm-Traktes erstellt.

Die durchgeführten Untersuchungen zur Einwirkung des biologischen Aktivators auf Wasser, das Patienten tranken, mit dem Zimmerpflanzen sowie Samen landwirtschaftlicher Kulturen behandelt wurden, bestätigen, daß der Aktivator effektiv und positiv die Eigenschaften des Wassers, den menschlichen Organismus und Pflanzensamen beeinflußt.

Einfluß der Beschaffenheit des Minerals, des Schnittwinkels und des Verhältnisses der Bezugsflächen auf die Keimfähigkeit von Samen:

| Beispiele | Stoff, aus dem die aktivierenden Elemente bestehen | Schnittwinkel | Verhältnis der Gesamtfläche der aktivierenden Elemente zur Fläche der Unterlage | % der Keimfähigkeit der Samen |
|---|---|---|---|---|
| 1 | Marmor | 90° | 1 | 46 |
| 2 | Pontischer Kalkstein | 0° | 1 | 60 |
| 3 | dito | 30° | 1 | 55 |
| 4 | dito | 90° | 1 | 88 |
| 5 | dito | 90° | 0,75 | 84 |
| 6 | dito | 90° | 0,6 | 52 |
| 7 | Silizium (Prototyp) | - | - | 54 |

## Patentansprüche

1. Biologischer Aktivator, der eine Auswahl aktivierender Elemente verschiedener Form aus einem natürlichen Mineral enthält, **dadurch gekennzeichnet, daß** jedes aktivierende Element aus verschiedenfarbigem pontischem Kalkstein in Form eines Körpers ausgebildet ist, der durch zwei parallele Geraden gebildet wird, die eine Fläche mit einem Winkel von 90° zur Bezugslinie der geologischen Schicht des Minerals bilden, wobei die aktivierenden Elemente fest mit einer Unterlage aus einem Werkstoff verbunden sind, der kein Metall enthält.

2. Aktivator nach Anspruch 1, **dadurch gekennzeichnet, daß** das Verhältnis der Gesamtfläche der aktivierenden Elemente zur Fläche der Unterlage, auf der sie befestigt sind, gleich 0,75 - 1,0 beträgt

3. Aktivator nach Anspruch 1, **dadurch gekennzeichnet, daß** die auf einer Unterlage befestigten aktivierenden Elemente in einem durchsichtigen Gehäuse angeordnet sind.
